# EUROPEAN PATENT APPLICATION

(11) **EP 0 716 887 A1**
(43) Date of publication of application: **19.06.1996**
(21) Application number: 94309275.9
(22) Date of filing: 12.12.1994
(51) Int. Cl.: B01J 38/00, B01J 29/90, B01J 27/28, C07C 2/18, C07C 2/12

(54) **Reactivation of used solid catalysts and use of the regenerated catalysts**

(71) Applicant: EXXON CHEMICAL PATENTS INC., Linden New Jersey 07036 (US)
(72) Inventor: De Meyer, Johan, M., B-2900 Schoten (BE); Godsmark, John, S., B-1390 Grez Doiceau (BE)
(74) Representative: Darby, David Thomas

(57) **Abstract**

A fouled solid catalyst is reactivated or rejuvenated by subjecting it to sub-atmospheric pressure at a temperature of at least 100°C.

The regeneration process is especially applicable to a solid phosphoric acid or to a zeolite particulate catalyst used in the form of a fixed bed.

The reactivated catalyst is used for the treatment of a hydrocarbon feedstock, such as olefin oligomerization.

## Description

This invention relates to catalysts, and especially to catalyst treatment. More especially, it is concerned with reactivation of used catalysts, in particular of solid phosphoric acid catalysts.

It is well known that a catalyst used in hydrocarbon conversion has a limited useful life. During operation, there is a build up in and on the catalyst of polymeric and carbonaceous by-products of the conversion reaction. This results both in a greater pressure drop across the catalyst bed, with increased operating expense, and lower catalyst activity as a result of the reduced effective active surface area of the catalyst.

Depending on the cost of the catalyst, a spent catalyst may be regenerated or discarded. However, since it is becoming increasingly difficult to dispose of spent catalyst in an environmentally acceptable way, attention has been given to regeneration of catalysts of all types, and to treatment of used, but not necessarily spent, catalysts to extend their useful life.

Solid phosphoric acid and other solid catalysts are much used in the petrochemical industry to effect numerous reactions. These include, for example, the oligomerization or hydrogenation of olefins, and their reaction with aromatic hydrocarbons or phenols. A solid phosphoric acid catalyst is normally formed by mixing a phosphorus acid, e.g., ortho-, pyro-, meta-, or poly-phosphoric acid, with a solid carrier to form a paste. The carrier may, for example, be a synthetic or natural porous silica or other oxide-containing material, e.g., keiselguhr, kaolin, infusorial earth, diatomaceous earth, activated clay, a zeolite, or an oxide of aluminium, zirconium, titanium or thorium. The paste may then be calcined and the resulting mass crushed or the paste may be extruded and pelletized and then calcined to yield uniform catalyst particles.

The catalyst may contain other components, for example, mineral talc, fuller's earth, and various metals or their oxides, e.g., nickel, copper, cobalt, zinc, manganese and, especially, iron, to modify its physical properties, e.g., thermal conductivity, strength and attrition resistance.

It has been proposed to regenerate or reactivate catalysts by a variety of treatments. Some of the regeneration proposals amount virtually to preparing new solid catalyst, while others themselves generate a product with disposal difficulties.

In U.S. Patent No. 3823085, there is disclosed a process for reactivating a supported sulphided metal oxide catalyst that is being used for catalytically hydrogenating low grade petroleum oils by reducing reactor pressure while continuing reactant flow through the reactor. In U.S. Patent No. 4062801, a solid phosphoric acid catalyst that has been used for catalysing an olefin oligomerization reaction is regenerated by terminating the flow of reactant, reducing reactor pressure to a minimum of 10 psig, or about 70 kPa gauge, introducing an aromatic solvent, repressurizing the reactor, removing the solvent and depressurizing, and repeating the cycle at least twice.

The procedure of U.S. Patent No. 3823085 results, it is stated, in a poor quality product being produced during the period of reduced pressure (stated in the patent to be a minimum 100 psig, or about 700 kPa gauge) since the coloured polymeric material from the catalyst is drawn off in the product. While this is not a problem with the procedure of U.S. Patent No. 4062801, that is time-consuming and complex, and produces a large quantity of contaminated solvent.

There remains a need, therefore, for a catalyst reactivating procedure that mitigates these difficulties.

The present invention is based on the observation that a catalyst may be reactivated by subjecting it to a sub-atmospheric pressure.

The invention accordingly provides a process for the reactivation of a solid catalyst that has been used for catalysing a reaction that causes build up of hydrocarbon or carbonaceous deposits on the catalysts, the reaction taking place at a pressure at least equal to atmospheric pressure, which comprises subjecting the catalyst to a sub-atmospheric pressure.

The reactor pressure may be reduced by evacuation through a line, referred to below as an evacuator line, which may be a line normally used during the reaction as an inlet to or outlet from the reactor, or one provided for evacuation purposes only. The evacuated materials may be removed from the reactor into the downstream system to minimize product loss.

The catalyst is advantageously a particulate catalyst, and is preferably used in the form of a fixed bed. The bed may be of any configuration, and there may be more than a single bed, in which case the beds may be evacuated separately, so that material evacuated from one bed is not drawn through another. The catalyst may be contained in one or more reactors, which may, for example, be chamber or tubular reactors.

The reaction catalysed by the catalyst to be reactivated may be, for example, a hydrocarbon conversion, e.g., olefin oligomerization or hydrogenation, reaction of an olefin with an aromatic moiety, the preparation of tertiary olefins from a tertiary ether (e.g., isobutylene from methyl t-butyl ether), the hydrotreating of heavy mineral or shale oil or other feedstocks, the cracking of mono- or dialkyl dioxolanes to conjugated diolefins (e.g., dimethyl dioxolane to isoprene), the cyclization of alkenylbenzenes to dialkyltetralins, and dehydrations, e.g., of butanediol to butadiene. For example, propene or butenes may be caused to form dimers, trimers and tetramers or hydrogenated to form propane or butanes, and olefins and aromatic hydrocarbons or phenols reacted to produce, for example, ethylbenzene, cumene, or thymols. All these reactions tend to produce higher molecular weight materials as by-products, and these may be retained in or on the catalyst.

The reactions generally take place at pressures within the range of from 0.1 to 10 MPa gauge, more usually from 0.75 to 7.5 MPa, and especially from 1.5 to 2 MPa.

The present invention also provides a process for the treatment of an organic feedstock, especially a hydrocarbon feedstock, and more especially a feedstock comprising an olefin component, at a pressure at least equal to atmospheric, in the presence of a solid catalyst, especially a solid phosphoric acid catalyst, in which the process is interrupted at least once to subject the catalyst to a sub-atmospheric pressure.

The life of a catalyst is limited by a number of factors; one limitation is the build up of pressure drop in the reactor. The process of the invention mitigates this effect; and the greater the pressure drop, the greater is the reduction in pressure drop achieved by the inventive process.

The catalytic reactions generally take place at temperatures within the range of from 100° to 145°C, more usually from 120° to 375°C, and especially from 200°C to 300°C, depending on the reactants. Depressurizing according to the invention may be effected at similar temperatures, at least initially, although the temperature may be allowed to fall while sub-atmospheric pressure is maintained. Advantageously, however, the temperature is maintained above 100°C, preferably above 150°C, and advantageously above 175°C, by, for example, maintaining the temperature of the reactor heating means, e.g., the water in the shell of a tubular reactor.

Pressure is advantageously reduced to a value of at most 20 kPa (absolute), and preferably to a value within the range 60 to 20 kPa, most preferably about 30 kPa.

Removal of material from the catalyst may be assisted by introducing an inert gas, e.g., nitrogen, into the reactor while evacuating.

The pressure is advantageously initially lowered by evacuation from the bottom only, to minimize lifting of the bed. The reduced pressure is advantageously maintained for a period within the range of from 30 minutes to 5 hours, preferably from 1 to 3 hours, and most preferably for about 3 hours. The process of the invention may be repeated whenever the pressure drop through the reactor reaches a predetermined level, e.g., 100 kPa, or at intervals, e.g., of from 1 to 10 days, advantageously from 4 to 8 days, and preferably every 7 days, during the catalyst's life.

Although we do not wish to be bound by any theory, it is believed that, when a reactor is evacuated, in addition to feed and product present in the reactor there is also removed one or more of heavy hydrocarbon (e.g., polymer) or carbonaceous (e.g., tar) deposits, and water. If the materials removed include water, the reduction in pressure drop may be attributed to an increase in void content.

In any event, whatever the mode of action of the process, its efficacy is apparent from the reduction in the pressure drop, or the reduction in its rate of increase, seen immediately after the process. Pressure drop reduction may result in an improvement in catalyst life of up to 30%.

The invention is especially applicable to particulate catalysts, and more especially to solid phosphoric acid and zeolites.

### Example

Two propene oligomerization reactors of the vertical tubular type with a top inlet and bottom outlet were filled with solid phosphoric acid catalyst. One reactor (R1) was operated as a control, and a second reactor (R2) was operated under similar reaction conditions, about 6 MPa gauge and 200°C. The process of the invention was carried out at approximately weekly intervals in reactor R2.

The procedure of the invention comprised reducing the feed rate to the second reactor and then blocking the reactor by closing the inlet and outlet valves. The reactor was depressurized by opening the outlet evacuator line; after full depressurization, the inlet evacuator line was opened; a sub-atmospheric pressure of about 33 kPa absolute was maintained for 3 hours. After this period, the reactor was started up again.

The Table below shows the pressure drop build-up for each day of operation of the two reactors, and the days on which the process of the invention was carried out on the second reactor. Each reactor was taken offline when the pressure drop reached about 300 kPa.

**Table**

| Day | Pressure Drop, kPa | | Day | Pressure Drop, kPa | |
|---|---|---|---|---|---|
| | R1 | R2 | | R1 | R2 |
| 1 | 3 | 6 | 31* | 270 | 109 |
| 7 | 11 | 7 | 32 | 294 | 112 |
| 8* | 13 | 8 | 33 | 318 | 120 |
| 9 | 14 | 9 | 36 | -- | 159 |
| 15 | 32 | 18 | 37* | -- | 166 |
| 16* | 38 | 20 | 38 | -- | 174 |
| 17 | 43 | 22 | 42 | -- | 255 |
| 22 | 99 | 41 | 43* | -- | 260 |
| 23* | 115 | 45 | 44 | -- | 266 |
| 24 | 131 | 49 | 46 | -- | 301 |

| | | | | | |
|---|---|---|---|---|---|
| * Depressurization on these days. | | | | | |

The Control Reactor was on line for 33 days, and achieved a catalyst life of 488 wt/wt oligomer/catalyst; the second reactor was on line for 46 days and achieved a catalyst life of 574 wt/wt oligomer/catalyst, an improvement of 18%.

## Claims

1. A process for the reactivation of a solid catalyst that has been used for catalysing a reaction that results in hydrocarbon or carbonaceous deposition on the catalyst, the reaction taking place at a pressure at least equal to atmospheric pressure, which comprises subjecting the catalyst to a sub-atmospheric pressure.

2. A process as claimed in claim 1, wherein the catalyst is subjected to a pressure within the range of 20 kPa (absolute) to atmospheric, advantageously from 20 to 60 kPa (absolute).

3. A process as claimed in claim 1 or claim 2, wherein the catalyst is maintained at sub-atmospheric pressure for from 30 minutes to 5 hours, advantageously for from 1 to 3 hours.

4. A process as claimed in any one of claims 1 to 3, wherein the catalyst is subjected to sub-atmospheric pressure at a temperature of at least 100°C, advantageously at least 150°C and preferably at least 175°C.

5. A process as claimed in any one of claims 1 to 4, wherein the catalyst is solid phosphoric acid or a zeolite, advantageously in the form of a fixed bed, advantageously in particulate form, and advantageously in a chamber or tubular reactor.

6. A process for the treatment of an organic feedstock at a pressure at least equal to atmospheric pressure, in the presence of a solid catalyst, in which the treatment is interrupted at least once to subject the catalyst to a sub-atmospheric pressure, advantageously under the conditions specified in any one of claims 2 to 5.

7. A process as claimed in claim 6, wherein the catalyst is subjected to sub-atmospheric pressure at intervals, for example at from 1 to 10 days, advantageously from 4 to 8 days, or whenever the pressure drop through the catalyst reaches a predetermined value.

8. A process as claimed in claim 6 or claim 7, which comprises the treatment of a hydrocarbon feedstock, more especially an olefin feedstock, the treatment preferably comprising olefin oligomerization.

9. Any new feature described herein or any new combination of hereindescribed features.
